# EUROPÄISCHE PATENTSCHRIFT

(11) **EP 3 800 480 B1**
(45) Veröffentlichungstag und Bekanntmachung des Hinweises auf die Patenterteilung: **29.11.2023**
(21) Anmeldenummer: 19200780.5
(22) Anmeldetag: 01.10.2019
(51) Int. Cl.: G01R 33/54, A61B 5/055

(54) **KONVERTIERUNG EINES MR-MESSPROTOKOLLS**
MR MEASUREMENT PROTOCOL CONVERSION
CONVERSION D'UN PROTOCOLE DE MESURE MR

(43) Veröffentlichungstag der Anmeldung: 07.04.2021
(73) Patentinhaber: Siemens Healthcare GmbH, 91052 Erlangen (DE)
(72) Erfinder: Meyer, Stefan, 91094 Langensendelbach (DE)

(56) Entgegenhaltungen:
- DE-A1-102016 207 848
- US-A1- 2006 271 925
- US-A1- 2014 348 401
- US-A1- 2018 068 070
- US-A1- 2018 306 883

## Beschreibung

Die vorliegende Erfindung betrifft die Konvertierung eines MR-Messprotokolls, welches Parameter mit zugehörigen Parameterwerten vorgibt, die eine Messung mittels einer Magnetresonanzanlage konfigurieren.

Bei Magnetresonanzanlagen bzw. MR-(Magnetresonanz-) Tomographen wird die Gesamtheit aller Parameter, welche die bildgebende Messung mittels der Magnetresonanzanlage beeinflussen bzw. konfigurieren, in MR-Messprotokollen oder Parameter-Sets abgelegt. Üblicherweise werden solche MR-Messprotokolle mit dem Rollout einer bestimmten Softwareversion mitgeliefert, welche auf die entsprechende Magnetresonanzanlage und den aktuellen Stand der Technik optimal abgestimmt ist.

Darüber hinaus haben klinische Benutzer der Magnetresonanzanlage auch die Möglichkeit, selbst MR-Messprotokolle (sogenannte Nutzerprotokolle) für ihre Messungen zu erstellen und abzuspeichern. Falls eine Magnetresonanzanlage auf eine neue Softwareversion aktualisiert wird, werden diese Nutzerprotokolle konvertiert, um sicherzustellen, dass sie mit der aktuellen Softwareversion auch konsistente Parametereinstellungen aufweisen.

Bei Benutzern von Magnetresonanzanlagen, die mit zahlreichen Magnetresonanzanlagen arbeiten, sind bisweilen unterschiedliche Softwareversionen für gleiche oder auch verschiedene Magnetresonanzanlagen im Einsatz. Bei diesen Benutzern existiert der Wunsch, ein MR-Messprotokoll, welches für irgendeine Softwareversion erzeugt wurde, auch für alle anderen Softwareversionen, die auf irgendwelchen Magnetresonanzanlagen laufen, einsetzen zu können.

DE 10 2016 207 848 A1 beschreibt ein Verfahren zum Erzeugen eines Messprotokolls für eine medizinische Bildgebung von einem zu untersuchenden Objekt. Bei dem Verfahren werden Messparameter des Messprotokolls in eine Protokollstruktur mit einer Basisklasse und die Basisklasse ergänzenden Klassen eingeteilt, wobei die Basisklasse hardware-unabhängige Messparameter und die ergänzenden Klassen hardwarespezifische Messparameter umfassen.

US 2018 306 883 A1 beschreibt ein Verfahren zum Betrieb einer Bildaufnahmeeinrichtung welche einen Aufnahmevorgang definierende Aufnahmeprotokolle aufweist, wobei bei einer den möglichen Einstellungsraum für Aufnahmeparameter verändernden Aktualisierung wenigstens einer Software- und/oder Hardwarekomponente der Bildaufnahmeeinrichtung auf eine aktuelle Ausstattungsversion und/oder bei Eingang eines nicht für die aktuelle Ausstattungsversion definierten Aufnahmeprotokolls ein Regelsatz auf alle nicht für die aktuelle Ausstattungsversion definierten Protokolle angewendet wird.

US 2018 068 070 A1 beschreibt ein Verfahren zu einer Bestimmung eines Ähnlichkeitsparameters für ein Ursprungsprotokoll mit einem Referenzprotokoll, wobei das Ursprungsprotokoll Steuerparameter zur Ansteuerung eines medizinischen Bildgebungsgerätes umfasst und mit einer Ursprungskonfiguration des medizinischen Bildgebungsgerätes kompatibel ist und das Referenzprotokoll Steuerparameter zur Ansteuerung eines medizinischen Bildgebungsgerätes umfasst und mit einer Neukonfiguration kompatibel ist, wobei der Ähnlichkeitsparameter mittels einer Vergleichsanalyse für das Ursprungsprotokoll mit dem Referenzprotokoll bestimmt wird.

US 2006 271 925 A1 beschreibt ein Verfahren zu einem Aktualisieren von Software in einem Remote-Service-Center. Dabei werden kundenspezifischen Daten bezüglich einer früheren Version einer Softwareanwendung von einem Kundenstandort in einem Remote-Service-Center empfangen und eine überarbeitete Version der Softwareanwendung im Remote-Service-Center modifiziert.

US 2014 348 401 A1 beschreibt eine Bildverarbeitungsvorrichtung mit einer Scanprotokoll-Erstellungseinheit, die konfiguriert ist, um ein Scanprotokoll für einen bestimmten Teil des Körpers eines aktuell zu scannenden Objekts zu erstellen. Daher stellt sich die vorliegende Erfindung die Aufgabe, dafür zu sorgen, dass ein MR-Messprotokoll, welches für eine neue Softwareversion erzeugt wird, auch für eine alte Softwareversion einer Magnetresonanzanlage einsetzbar ist.

Erfindungsgemäß wird diese Aufgabe durch ein Verfahren zur Konvertierung eines MR-Messprotokolls nach Anspruch 1, durch eine Vorrichtung zur Konvertierung eines MR-Messprotokolls nach Anspruch 8, durch eine Magnetresonanzanlage nach Anspruch 10, durch ein Computerprogrammprodukt nach Anspruch 11 und durch einen elektronisch lesbaren Datenträger nach Anspruch 12 gelöst. Die abhängigen Ansprüche definieren bevorzugte und vorteilhafte Ausführungsformen der vorliegenden Erfindung.

Im Rahmen der vorliegenden Erfindung wird ein Verfahren zur Konvertierung eines MR-Messprotokolls bereitgestellt. Dabei gibt ein MR-Messprotokoll Parameter mit zugehörigen Parameterwerten vor, durch welche eine Messung mittels einer neuen Softwareversion durch eine erste Magnetresonanzanlage konfiguriert wird. Das erfindungsgemäße Verfahren umfasst folgende Schritte:
- Syntaktisches Konvertieren des Messprotokolls. Dabei wird die Struktur des MR-Messprotokolls automatisch an eine alte Softwareversion einer zweiten Magnetresonanzanlage angepasst.
- Semantisches Konvertieren des syntaktisch konvertierten MR-Messprotokolls. Bei dieser semantischen Konvertierung werden Abhängigkeiten der Parameterwerte bestimmter Parameter hinsichtlich der alten Softwareversion automatisch berücksichtigt. In diesem Schritt wird insbesondere überprüft, ob die Parameterwerte des syntaktisch konvertierten MR-Messprotokolls innerhalb der entsprechenden Applikation noch semantisch korrekt sind. Falls dies nicht der Fall ist, werden die Parameterwerte entsprechend automatisch geändert.

Die semantische Konvertierung passt insbesondere den Inhalt der MR-Messprotokolle (d.h. die Parameterwerte) auf die in der alten Softwareversion implementierten Eigenschaften an, indem die Abhängigkeiten zwischen den Parametern einschließlich ihrer Parameterwerte berücksichtigt werden.

Indem ein für eine neue Softwareversion geschriebenes MR-Messprotokoll durch die erfindungsgemäße Konvertierung auch an eine alte Softwareversion angepasst wird, wird oben gestellte Aufgabe gelöst. Neben der bekannten Konvertierung, bei welcher MR-Messprotokolle von älteren Softwareversionen auf neuere Softwareversionen konvertiert werden, ist es erfindungsgemäß nunmehr auch möglich, ein MR-Messprotokoll einer neueren Softwareversion auf eine ältere Softwareversion zu konvertieren. Dadurch ist es vorteilhafterweise möglich, ein MR-Messprotokoll einer neueren Softwareversion auch auf einer Magnetresonanzanlage mit einer älteren Softwareversion zu importieren und zu benutzen. Damit können Benutzer ein MR-Messprotokoll, welches für eine bestimmte Softwareversion geschrieben ist, auch für andere Softwareversionen einsetzen, unabhängig davon ob es sich bei dieser Softwareversion um eine neuere oder ältere Softwareversion handelt.

Unter einer neuen Softwareversion wird dabei insbesondere eine Softwareversion verstanden, welche zeitmäßig nach der alten Softwareversion erstellt wurde und beispielsweise Software-Merkmale aufweist, welche die alte Softwareversion nicht aufweist. Die neue Softwareversion ist insbesondere an den zum Zeitpunkt ihrer Erstellung aktuellen Stand der Technik angepasst, der neuer ist als der Stand der Technik, der bei der Erstellung der alten Softwareversion galt.

Zur erfindungsgemäßen Konvertierung des MR-Messprotokolls werden insbesondere Konvertierregeln vorgegeben. Dabei passt jede dieser Konvertierregeln einen Parameter des MR-Messprotokolls an die alte Softwareversion an. Diese Anpassung eines Parameters kann dabei ein Umbenennen und ein Löschen dieses Parameters und/oder ein Ändern des Parameterwertes dieses Parameters umfassen. Diese Konvertierregeln werden insbesondere bei dem Schritt der syntaktischen Konvertierung eingesetzt, wobei sie beispielsweise (nur) ein Suchen und Ersetzen (Search- & Replace-Funktionalität) bezüglich der Parameter realisieren.

Diese Konvertierregeln können beispielsweise zusammen mit MR-Messprotokollen für die neue Softwareversion gespeichert werden. Anders ausgedrückt werden die Konvertierregeln für die Konvertierung von der neuen auf die alte Softwareversion zusammen mit dem MR-Messprotokoll exportiert und mit dem (neuen) MR-Messprotokoll abgelegt.

Indem die Konvertierregeln zusammen mit den MR-Messprotokollen für die neue Softwareversion gespeichert werden, ist sichergestellt, dass die Konvertierregeln vorliegen, wenn die MR-Messprotokolle für die neue Softwareversion auf die ältere Softwareversion konvertiert werden.

Erfindungsgemäß ist es allerdings auch möglich, die syntaktische Konvertierung ohne Konvertierregeln durchzuführen. Das führt insbesondere dazu, dass gemäß der alten Softwareversion unbekannte Parameter im MR-Messprotokoll nicht eingelesen werden können und dass Parameter, welche laut der alten Softwareversion im MR-Messprotokoll erwartet werden, aber nicht vorhanden sind, neu initialisiert werden. Beide vorab skizzierten Fälle führen beispielsweise dazu, dass diese Parameter im syntaktisch konvertierten MR-Messprotokoll mit Default-Werten initialisiert werden. Beispielsweise dadurch auftretende Inkonsistenzen können dann in der folgenden semantischen Konvertierung aufgelöst werden.

Der Schritt der semantischen Konvertierung wird insbesondere unabhängig davon, ob die syntaktische Konvertierung mit oder ohne Konvertierregeln arbeitet, gleich ausgeführt.

Beide Möglichkeiten (syntaktische Konvertierung mit oder ohne Konvertierregeln) führen zu einer erfolgreichen Konvertierung des MR-Messprotokolls auf die ältere Softwareversion.

Bei der ersten Magnetresonanzanlage kann es sich um die zweite Magnetresonanzanlage handeln, was bedeutet, dass die erste und die zweite Magnetresonanzanlage dieselbe Magnetresonanzanlage sind. Es ist auch möglich, dass die erste und die zweite Magnetresonanzanlage vom selben Magnetresonanzanlagen-Typ sind. In diesen Fällen gelten die neue und die alte Softwareversion für die gleiche Hardware bzw. den gleichen Typ einer Magnetresonanzanlage.

Es ist aber erfindungsgemäß auch möglich, dass sich die erste Magnetresonanzanlage bezüglich ihrer Hardware von der zweiten Magnetresonanzanlage unterscheidet, so dass die erste Magnetresonanzanlage hinsichtlich ihres Herstellungsdatums neuer als die zweite Magnetresonanzanlage ist. In diesem Fall ist die neue Softwareversion insbesondere an die Hardware der ersten Magnetresonanzanlage angepasst, während die alte Softwareversion an die Hardware der zweiten Magnetresonanzanlage angepasst ist, welche sich von der Hardware der ersten Magnetresonanzanlage unterscheidet.

Das syntaktische Konvertieren umfasst insbesondere folgende Schritte:
- Entfernen von Parametern des MR-Messprotokolls, welche von der alten Softwareversion nicht unterstützt werden.
- Einfügen von neuen Parametern des MR-Messprotokolls, welche zwar von der alten Softwareversion, aber nicht von der neuen Softwareversion unterstützt werden.
- Setzen der neuen Parameter auf einen Standardwert.
- Übernehmen von Parameterwerten von Parametern des MR-Messprotokolls, welche bei der alten Softwareversion einen anderen Namen haben als bei der neuen Softwareversion.

Das semantische Konvertieren umfasst insbesondere folgende Schritte:
- Erzeugen eines Standard-Protokolls der alten Softwareversion als ein Ziel-Protokoll.
- Kopieren von Parametern des MR-Messprotokolls in die zugehörigen Parameter des Ziel-Protokolls. Genauer gesagt werden in diesem Schritt Parameterwerte der Parameter des MR-Messprotokolls den zugehörigen Parametern des Ziel-Protokolls zugewiesen. Dabei werden Grenzen und Abhängigkeiten der Parameter des Ziel-Protokolls bezüglich der alten Softwareversion berücksichtigt.

Am Ende des semantischen Konvertierens sollte das Ziel-Protokoll dem hinsichtlich der alten Softwareversion konvertierten MR-Messprotokoll entsprechen.

Bei den vorab skizzierten Schritten des semantischen Konvertierens werden insbesondere mehrfach alle Parameter des Ziel-Protokolls dahingehend überprüft, ob die Parameterwerte dieser Parameter die Grenzen und Abhängigkeiten bezüglich der alten Softwareversion einhalten. Falls dies nicht der Fall ist (d.h. zumindest einer der Parameterwerte der Parameter des Ziel-Protokolls verletzt eine der Grenzen oder eine der Abhängigkeiten bezüglich der alten Softwareversion), werden die Parameterwerte der Parameter des Ziel-Protokolls so gesetzt, dass die Parameterwerte oder Parameter die Grenzen und Abhängigkeiten bezüglich der alten Softwareversion nicht mehr verletzen. Dies wird insbesondere so lange wiederholt, bis alle Parameterwerte die Grenzen und Abhängigkeiten bezüglich der alten Softwareversion einhalten.

Im Rahmen der vorliegenden Erfindung wird auch eine Vorrichtung zur Konvertierung eines MR-Messprotokolls bereitgestellt. Dabei umfasst die erfindungsgemäße Vorrichtung eine Recheneinheit und Speichermittel. Die Vorrichtung ist ausgestaltet, um das MR-Messprotokoll vollständig oder teilweise in den Speichermitteln zu speichern, um mit Hilfe der Recheneinheit das MR-Messprotokoll syntaktisch zu konvertieren, und um mittels der Recheneinheit das syntaktisch konvertierte MR-Messprotokoll semantisch zu konvertieren, indem Abhängigkeiten der Parameterwerte bestimmter Parameter bezüglich der alten Softwareversion berücksichtigt werden.

Die Vorteile der erfindungsgemäßen Vorrichtung entsprechen im Wesentlichen den Vorteilen des erfindungsgemäßen Verfahrens, welche vorab im Detail ausgeführt sind, so dass hier auf eine Wiederholung verzichtet wird.

Darüber hinaus wird eine erfindungsgemäße Magnetresonanzanlage offenbart, welche die vorab beschriebene erfindungsgemäße Vorrichtung umfasst.

Des Weiteren beschreibt die vorliegende Erfindung ein Computerprogrammprodukt, insbesondere eine Software, welche man in einen Speicher einer programmierbaren Steuereinrichtung bzw. einer Recheneinheit einer Magnetresonanzanlage laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuereinrichtung läuft. Dabei benötigt das Computerprogrammprodukt eventuell Programmmittel, z.B. Bibliotheken und Hilfsfunktionen, um die entsprechenden Ausführungsformen des Verfahrens zu realisieren. Mit anderen Worten soll mit dem auf das Computerprogrammprodukt gerichteten Anspruch insbesondere eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert und gebunden oder der nur interpretiert werden muss, oder um einen ausführbaren Softwarecode handeln, der zur Ausführung nur noch in die entsprechende Recheneinheit bzw. Steuereinrichtung zu laden ist.

Schließlich offenbart die vorliegende Erfindung einen elektronisch lesbaren Datenträger, z.B. eine DVD, ein Magnetband, eine Festplatte oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuereinrichtung bzw. Recheneinheit einer Magnetresonanzanlage gespeichert werden, können alle erfindungsgemäßen Ausführungsformen des vorab beschriebenen Verfahrens durchgeführt werden.

Vorteilhafterweise können MR-Messprotokolle, welche für neue Softwareversionen konzipiert sind, auch auf Magnetresonanzanlagen importiert und verwendet werden, welche mit einer älteren Softwareversion arbeiten. Dadurch wird einem Benutzer dieser Magnetresonanzanlage mit älterer Software die Arbeit erspart, dass entsprechende MR-Messprotokoll bezüglich der alten Software neu anlegen zu müssen.

Die vorliegende Erfindung ermöglicht somit vorteilhafterweise eine Rückwärtskompatibilität von MR-Messprotokollen.

Benutzern, bei welchen zahlreiche Magnetresonanzanlagen mit unterschiedlichen Softwareversionen im Einsatz sind, ermöglicht die vorliegende Erfindung, dass ein MR-Messprotokoll von jeder beliebigen Magnetresonanzanlage exportiert und auf alle anderen Magnetresonanzanlagen importiert werden kann.

Im Folgenden wird die vorliegende Erfindung anhand erfindungsgemäßer Ausführungsformen mit Bezug zu den Figuren im Detail beschrieben.

In Fig. 1 ist der Ablauf einer erfindungsgemäßen Konvertierung dargestellt.

Fig. 2 stellt eine erfindungsgemäße Magnetresonanzanlage dar.

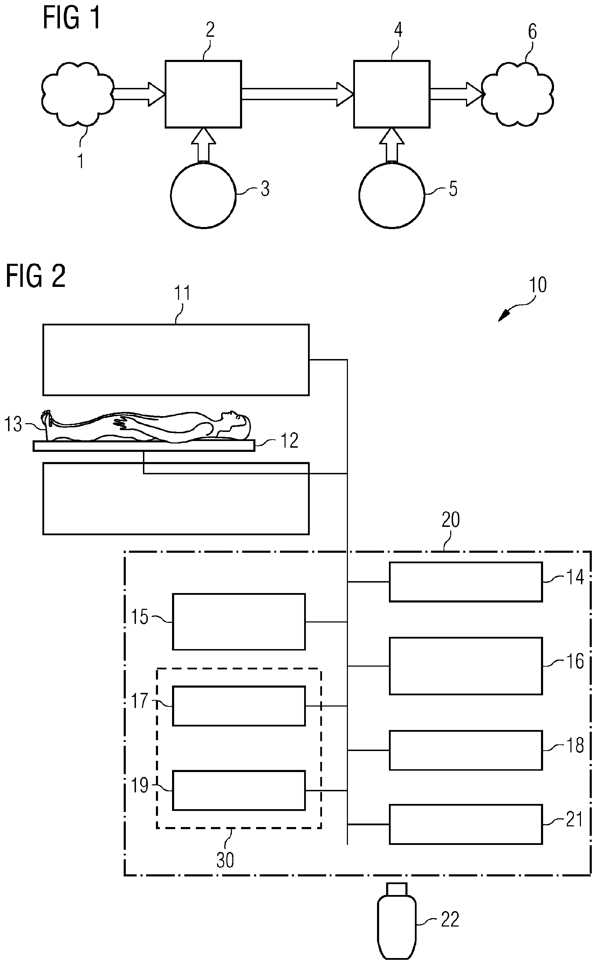

In Fig. 1 ist der Ablauf einer erfindungsgemäßen Konvertierung dargestellt, bei welcher ein MR-Messprotokoll 1 einer neuen Softwareversion in ein MR-Messprotokoll 6 einer alten Softwareversion konvertiert wird.

In einem ersten Schritt erfolgt eine syntaktische Konvertierung 2 des MR-Messprotokolls der neuen Softwareversion unter Verwendung von Konvertierregeln 3. Optional kann dieser erste Schritt auch ohne Konvertierregeln 3 durchgeführt werden.

In einem zweiten Schritt wird dann das syntaktisch konvertierte MR-Messprotokoll einer semantischen Konvertierung 4 unterzogen, wobei Parameterabhängigkeiten 5 berücksichtigt werden. Das Ergebnis dieses zweiten Schrittes ist das konvertierte MR-Messprotokoll 6 für die alte Softwareversion.

Die in Fig. 1 skizzierte automatisch ablaufende erfindungsgemäße Konvertierung soll im Folgenden genauer skizziert werden.

Zuerst wird die Datenstruktur des zu konvertierenden MR-Messprotokolls an die Datenstruktur der alten Softwareversion angepasst. Während dieser Anpassung werden in der alten Softwareversion nicht mehr vorhandene Parameter entfernt, in der alten Softwareversion, aber nicht in der neuen Softwareversion vorhandene Parameter eingeführt und auf einen Default-Wert gesetzt und Parameterwerte für umbenannte Parameter (d.h. Parameter, die in der alten Softwareversion einen anderen Namen tragen als in der neuen Softwareversion, aber dieselbe Funktionalität aufweisen) übernommen. Wenn Sequenzen in der alten Softwareversion ersetzt werden, dann wird ein Verweis auf die Sequenz, welche von dem konvertierten Protokoll eingesetzt wird, ebenfalls aktualisiert.

Anschließend wird ein Default-MR-Messprotokoll der Sequenz, welche von dem konvertierten MR-Messprotokoll eingesetzt wird, als neues (Ziel-) MR-Messprotokoll erzeugt, und es wird versucht, den Spulenkontext (coil context) des zu konvertierenden MR-Protokolls auf das Ziel-MR-Messprotokoll anzuwenden. Wenn Spulen, welche in dem zu konvertierenden MR-Messprotokoll ausgewählt sind, in der alten Softwareversion (z.B. einer alten Magnetresonanzanlage) nicht verfügbar sind, wird eine kompatible Spule bezüglich der alten Softwareversion (z.B. der alten Magnetresonanzanlage) gesucht, und es wird versucht, die entsprechenden Spulenelemente einzustellen. Wenn keine kompatible Spule gefunden wird, wird die Körperspule verwendet. Dies kann zu Anpassungen von abhängigen Parametern (z.B. PAT-Faktor, Normalisierungsfilter eines Vorabscans) führen.

Schließlich wird jeder Parameterwert in mehreren Schleifen von dem zu konvertierenden MR-Messprotokoll in das Ziel-Messprotokoll kopiert, wobei Grenzen und Abhängigkeiten dieser Parameter bezüglich der alten Softwareversion beachtet werden. Bei jedem dieser Durchläufe können andere Parameter angepasst werden, um dadurch die Parameterabhängigkeiten der alten Softwareversion einzuhalten.

Am Ende der Konvertierung entspricht das Ziel-MR-Messprotokoll dem konvertierten MR-Messprotokoll für die alte Softwareversion.

Im Folgenden wird die Konvertierung eines MR-Messprotokolls von einer neuen Softwareversion auf eine alte Softwareversion anhand eines konkreten kurzen Beispiels im Detail erläutert.

**Tabelle 1: MR-Messprotokoll gemäß neuer Softwareversion**

| |
|---|
| converter=%MEASCONST%/ConverterList/Prot_Converter.txt ulVersion = 61010001 |
| tSequenceFileName = "%SiemensSeq%\space" |
| tProtocolName = "t2_spc_rst_cor_p3_trig_384_iso" tdefault- |
| EVAProt = "%SiemensEvaDefProt%\Inline\Inline.evp" lMeasPause = 30000000 |
| alTR[0] = 2400000 |
| sKSpace.lBaseResolution = 384 |
| sKSpace.ucMultiSliceMode = 1 |
| sPat.ucPATMode = 2 |

**Tabelle 2: Konvertierregeln**

| |
|---|
| [61010002 -> 61010001] // VersionNeu -> VersionAlt |
| //Ersetzt die dahinterliegende Sequenz (==Applikation) if (tSequenceFileName == "%SiemensSeq%\space") set (tSequenceFileName, "%SiemensSeq%\space2") |
| //Entfernt den Parameter delete (lMeasPause) |
| //Umbenennen eines Parameters rename(alTR, alRepetitionTime) |
| //Ändern eines Parameterwertes in Abhängigkeit von anderen Parametern (z.B. Änderung durch ein Feature, das in der alten, aber nicht in der neuen Softwareversion existiert) |
| if (sKSpace.ucMultiSliceMode == 1) set |
| (sKSpace.lBaseResolution, 512) |
| //Setzen eines Parameterwertes (z.B. Einschalten eines Features, das in der alten, aber nicht in der neuen Softwareversion existiert) |
| if (sPat.ucPATMode == 2) set (sPat.ucPATMode, 4) |
| //Hinzufügen eines Parameters default(newParameter, 42) |

**Tabelle 3: in alte Softwareversion konvertiertes MR-Messprotokoll**

| |
|---|
| tSequenceFileName = "%SiemensSeq%\space2" |
| tProtocolName = "t2_spc_rst_cor_p3_trig_384_iso" tdefaultEVA- |
| Prot = "%SiemensEvaDefProt%\Inline\Inline.evp" alRepetition- |
| Time[0] = 2400000 |
| sKSpace.lBaseResolution = 512 |
| sKSpace.ucMultiSliceMode = 1 |
| sPat.ucPATMode = 4 |
| newParameter = 42 |

In Tabelle 1 ist ein MR-Messprotokoll, welches in einer neuen Softwareversion geschrieben ist, dargestellt. Dieses MR-Messprotokoll soll mit Hilfe der in Tabelle 2 dargestellten Konvertierregeln auf eine alte Softwareversion konvertiert werden. Das Ergebnis dieser erfindungsgemäßen Konvertierung ist in Form des konvertierten MR-Messprotokolls in Tabelle 3 dargestellt.

In Fig. 2 ist eine erfindungsgemäße Magnetresonanzanlage 10 dargestellt, welche einen Magneten 11 zur Erzeugung eines Polarisationsfelds B0 aufweist, wobei eine auf einer Liege 12 angeordnete Untersuchungsperson 13 in den Magneten 11 gefahren wird, um dort ortskodierte Magnetresonanzsignale aus der Untersuchungsperson 13 aufzunehmen. Die zur Signalaufnahme verwendeten Spulen wie eine Ganzkörperspule oder Lokalspulen sind aus Übersichtlichkeitsgründen nicht dargestellt.

Die Magnetresonanzanlage 10 weist weiterhin eine Steuereinheit 20 auf, die zur Steuerung der Magnetresonanzanlage 10 verwendet werden kann. Die Steuerung 20 weist eine Gradientensteuereinheit 15 zur Steuerung und Schaltung der notwendigen Magnetfeldgradienten auf. Eine HF-Steuereinheit 14 ist für die Steuerung und Generierung der HF-Pulse zur Auslenkung der Magnetisierung vorgesehen. Eine Bildsequenzsteuerung 16 steuert die Abfolge der Magnetfeldgradienten und HF-Pulse und damit indirekt die Gradientensteuereinheit 15 und die HF-Steuereinheit 14. Über eine Eingabeeinheit 18 kann eine Bedienperson die Magnetresonanzanlage steuern, und auf einer Anzeigeeinheit 21 können MR-Bilder und sonstige zur Steuerung notwendigen Informationen angezeigt werden. Eine Recheneinheit 17 mit mindestens einer Prozessoreinheit (nicht gezeigt) und Speichermittel 19 sind vorgesehen zur Steuerung der verschiedenen Einheiten in der Steuereinheit 20. Weiterhin können in den Speichermitteln 19 beispielsweise Programmmodule bzw. Programme abgespeichert sein, die, wenn sie von der Recheneinheit 17 bzw. ihrer Prozessoreinheit ausgeführt werden, den Ablauf der Magnetresonanzanlage 10 steuern können. Die Recheneinheit 17 und die Speichermittel 19 sind Elemente einer erfindungsgemäßen Vorrichtung 30, welche somit Bestandteil der erfindungsgemäße Magnetresonanzanlage 10 ist.

Darüber hinaus ist ein Datenträger oder USB-Stick 22 dargestellt, auf welchem ein erfindungsgemäßes Softwarepaket bzw. Computerprogrammprodukt gespeichert ist.

## Patentansprüche

1. Verfahren zur Konvertierung eines MR-Messprotokolls (1), welches Parameter mit zugehörigen Parameterwerten vorgibt, welche eine Messung mittels einer neuen Softwareversion einer ersten Magnetresonanzanlage konfigurieren,
wobei das Verfahren folgende Schritte umfasst:
syntaktisches Konvertieren (2) des MR-Messprotokolls (1), wobei die Struktur des MR-Messprotokolls (1) an eine alte Softwareversion einer zweiten Magnetresonanzanlage (10) angepasst wird,
semantisches Konvertieren (4) des syntaktisch konvertierten MR-Messprotokolls, indem Parameterwerte des syntaktisch konvertierten MR-Messprotokolls auf die in der alten Softwareversion implementierten Eigenschaften angepasst werden, wobei die Abhängigkeiten zwischen den Parametern einschließlich ihrer Parameterwerte berücksichtigt werden.

2. Verfahren nach Anspruch 1,
**dadurch gekennzeichnet,**
**dass** in dem Schritt der syntaktischen Konvertierung Konvertierregeln (3) vorgegeben werden,
wobei jede der Konvertierregeln (3) einen Parameter des MR-Messprotokolls (1) an die alte Softwareversion anpasst.

3. Verfahren nach Anspruch 2,
**dadurch gekennzeichnet,**
**dass** die Konvertierregeln (3) zusammen mit MR-Messprotokollen für die neue Softwareversion gespeichert werden.

4. Verfahren nach einem der vorherigen Ansprüche,
**dadurch gekennzeichnet,**
**dass** die erste Magnetresonanzanlage gleich der zweiten Magnetresonanzanlage ist, oder
**dass** sich die erste Magnetresonanzanlage hinsichtlich ihrer Hardware von der zweiten Magnetresonanzanlage (10) unterscheidet, wobei die erste Magnetresonanzanlage hinsichtlich ihres Herstellungsdatums neuer als die zweite Magnetresonanzanlage ist.

5. Verfahren nach einem der vorhergehenden Ansprüche, **dadurch gekennzeichnet,**
**dass** das syntaktische Konvertieren (2) umfasst ein Entfernen von Parametern des MR-Messprotokolls (1), welche von der alten Softwareversion nicht unterstützt werden, ein Einfügen von neuen Parametern des MR-Messprotokolls (1), welche von der alten Softwareversion, aber nicht von der neuen Softwareversion unterstützt werden,
ein Setzen der neuen Parameter auf einen Standardwert, und ein Übernehmen von Parameterwerten von Parametern des MR-Messprotokolls (1), welche bei der alten Softwareversion einen anderen Namen haben als bei der neuen Softwareversion.

6. Verfahren nach einem der vorhergehenden Ansprüche, **dadurch gekennzeichnet,**
**dass** das semantische Konvertieren (4) umfasst ein Erzeugen eines Standard-Protokolls der alten Softwareversion als ein Ziel-Protokoll, und
ein Kopieren von Parametern des MR-Messprotokolls (1) in die zugehörigen Parameter des Ziel-Protokolls, wobei Grenzen und Abhängigkeiten der Parameter des Ziel-Protokolls bezüglich der alten Softwareversion berücksichtigt werden, und
**dass** das Ziel-Protokoll am Ende des Verfahrens dem bezüglich der alten Softwareversion konvertierten MR-Messprotokoll (6) entspricht,
wobei mehrfach alle Parameter des Ziel-Protokolls dahingehend überprüft werden, ob Grenzen und Abhängigkeiten bezüglich der alten Softwareversion eingehalten werden, und
**dass** in einem Fall, in welchem einer der Parameter des Ziel-Protokolls eine der Grenzen oder eine der Abhängigkeiten verletzt, die Parameter des Ziel-Protokolls so gesetzt werden, dass dieser Parameter die Grenze und die Abhängigkeit nicht mehr verletzt,
wobei der Schritt des Überprüfens, ob Grenzen und Abhängigkeiten bezüglich der alten Softwareversion eingehalten werden, und der Schritt des Neusetzens des Parameters, sofern der Parameter des Ziel-Protokoll eine der Grenzen oder eine der Abhängigkeiten verletzt, so lange wiederholt werden, bis alle Parameterwerte die Grenzen und Abhängigkeiten bezüglich der alten Softwareversion einhalten.

7. Vorrichtung zur Konvertierung eines MR-Messprotokolls (1), welches Parameter mit zugehörigen Parameterwerten vorgibt, welche eine Messung mittels einer neuen Softwareversion einer ersten Magnetresonanzanlage konfigurieren,
wobei die Vorrichtung (30) eine Recheneinheit (17) und Speichermittel (19) umfasst,
wobei die Vorrichtung (30) ausgestaltet ist,
um das MR-Messprotokoll (1) zumindest teilweise in den Speichermitteln (19) zu speichern,
um mittels der Recheneinheit (17) das MR-Messprotokoll (1) syntaktisch zu konvertieren (2), wobei die Struktur des MR-Messprotokolls (1) an eine alte Softwareversion einer zweiten Magnetresonanzanlage (10) angepasst wird,
um mittels der Recheneinheit (17) das syntaktisch konvertierte MR-Messprotokoll semantisch zu konvertieren (4), indem Parameterwerte des syntaktisch konvertierten MR-Messprotokolls auf die in der alten Softwareversion implementierten Eigenschaften angepasst werden, wobei die Abhängigkeiten zwischen den Parametern einschließlich ihrer Parameterwerte berücksichtigt werden.

8. Vorrichtung nach Anspruch 7,
**dadurch gekennzeichnet,**
**dass** die Vorrichtung (30) zur Durchführung des Verfahrens nach einem der Ansprüche 2 bis 6 ausgestaltet ist.

9. Magnetresonanzanlage (10) mit einer Vorrichtung (30) nach Anspruch 7 oder 8.

10. Computerprogrammprodukt, welches ein Programm umfasst und direkt in einen Speicher (19) einer programmierbaren Steuereinrichtung (20) einer Magnetresonanzanlage (10) ladbar ist, mit Programm-Mitteln, um alle Schritte des Verfahrens nach einem der Ansprüche 1-6 auszuführen, wenn das Programm in der Steuereinrichtung (20) der Magnetresonanzanlage (10) ausgeführt wird.

11. Elektronisch lesbarer Datenträger mit darauf gespeicherten elektronisch lesbaren Steuerinformationen, welche derart ausgestaltet sind, dass sie bei Verwendung des Datenträgers in einer Steuereinrichtung (20) einer Magnetresonanzanlage (10) das Verfahren nach einem der Ansprüche 1-6 durchführen.

## Claims

1. Method for converting an MR measuring protocol (1), which predetermines parameters with associated parameter values, which configure a measurement by means of a new software version of a first magnetic resonance system,
wherein the method comprises the following steps:
syntactic conversion (2) of the MR measuring protocol (1), wherein the structure of the MR measuring protocol (1) is adjusted to an old software version of a second magnetic resonance system (10),
semantic conversion (4) of the syntactically converted MR measuring protocol, by parameter values of the syntactically converted MR measuring protocol being adjusted to the properties implemented in the old software version, wherein the dependencies between the parameters including their parameter values are taken into account.

2. Method according to claim 1,
**characterised in that**
conversion rules (3) are predetermined in the step of syntactic conversion,
wherein each of the conversion rules (3) adjusts a parameter of the MR measuring protocol (1) to the old software version.

3. Method according to claim 2,
**characterised in that**
the conversion rules (3) are stored together with MR measuring protocols for the new software version.

4. Method according to one of the preceding claims, **characterised in that**
the first magnetic resonance system is identical to the second magnetic resonance system or
the first magnetic resonance system differs from the second magnetic resonance system (10) in respect of its hardware, wherein in respect of its date of manufacture, the first magnetic resonance system is newer than the second magnetic resonance system.

5. Method according to one of the preceding claims, **characterised in that**
the syntactic conversion (2) comprises
removing parameters of the MR measuring protocol (1) which are not supported by the old software version,
inserting new parameters of the MR measuring protocol (1), which are supported by the old software version but not by the new software version,
setting the new parameters to a standard value, and
taking over parameter values from parameters of the MR measuring protocol (1), which have a different name in the old software version than in the new software version.

6. Method according to one of the preceding claims, **characterised in that**
the semantic conversion (4) comprises
generating a standard protocol of the old software version as a target protocol and
copying parameters of the MR measuring protocol (1) into the associated parameters of the target protocol, wherein limits and dependencies of the parameters of the target protocol are taken into account with respect to the old software version and
at the end of the method the target protocol corresponds to the MR measuring protocol (6) which is converted with respect to the old software version,
wherein
all parameters of the target protocol are checked repeatedly to determine whether limits and dependencies are complied with with respect to the old software version, and
in the event that one of the parameters of the target protocol breaches one of the limits or one of the dependencies, the parameters of the target protocol are set so that this parameter no longer breaches the limit and the dependency, wherein the step of checking whether limits and dependencies are observed in respect of the old software version and the step of resetting the parameter, provided the parameter of the target protocol does not breach one of the limits or one of the dependencies, are repeated until all parameter values observe the limits and dependencies with respect to the old software version.

7. Device for converting an MR measuring protocol (1), which predetermines parameters with associated parameter values, which configure a measurement by means of a new software version of a first magnetic resonance system,
wherein the device (30) comprises a computer unit (17) and storage means (19),
wherein the device (30) is configured
to store the MR measuring protocol (1) at least partially in the storage means (19),
to syntactically convert (2) the MR measuring protocol (1) by means of the computer unit (17), wherein the structure of the MR measuring protocol (1) is adjusted to an old software version of a second magnetic resonance system (10),
to semantically convert the syntactically converted MR measuring protocol by means of the computer unit (17), by parameter values of the syntactically converted MR measuring protocol being adjusted to the properties implemented in the old software version, wherein the dependencies between the parameters including their parameter values are taken into account.

8. Device according to claim 7,
**characterised in that**
the device (30) is configured to carry out the method according to one of claims 2 to 6.

9. Magnetic resonance system (10) with a device (30) according to claim 7 or 8.

10. Computer program product, which comprises a program and can be loaded directly into a memory (19) of a programmable control device (20) of a magnetic resonance system (10), and has program means in order to perform all the steps of the method according to one of claims 1-6 when the program is executed in the control device (20) of the magnetic resonance system (10).

11. Electronically readable data storage medium with items of electronically readable control information stored thereon, which are configured such that, on use of the data storage medium in a control device (20) of a magnetic resonance system (10), they carry out the method according to one of claims 1-6.

## Revendications

1. Procédé de conversion d'un protocole (1) de mesure de résonnance magnétique, qui prescrit des paramètres, par des valeurs de paramètres leur appartenant, qui configure une mesure au moyen d'une nouvelle version logicielle d'une première installation de résonnance magnétique,
dans lequel le procédé comprend les stades suivants :
conversion (2) syntactique du protocole (1) de mesure de résonnance magnétique, dans lequel on adapte la structure du protocole (1) de mesure de résonnance magnétique à une ancienne version logicielle d'une deuxième installation (10) de résonnance magnétique,
conversion (4) sémantique du protocole de mesure de résonnance magnétique transformé syntactiquement, en adaptant des valeurs de paramètres du protocole de mesure de résonnance magnétique transformé aux propriétés mises en oeuvre dans l'ancienne version logicielle, dans lequel on prend en compte les dépendances entre les paramètres, y compris de leurs valeurs de paramètres.

2. Procédé suivant la revendication 1,
**caractérisé**
**en ce que**, dans le stade de la conversion syntactique, on prescrit des règles (3) de conversion,
dans lequel chacune des règles (3) de conversion adapte un paramètre du protocole (1) de mesure de résonnance magnétique à l'ancienne version logicielle.

3. Procédé suivant la revendication 2,
**caractérisé**
**en ce que** l'on met les règles (3) de conversion en mémoire ensemble avec les protocoles de mesure de résonnance magnétique pour la nouvelle version de logiciel.

4. Procédé suivant l'une des revendications précédentes, **caractérisé**
**en ce que** la première installation de résonnance magnétique est pareille à la deuxième installation de résonnance magnétique, ou **en ce que** la première installation de résonnance magnétique se distingue, en ce qui concerne son matériel, de la deuxième installation (10) de résonnance magnétique, dans lequel la première installation de résonnance magnétique est, en ce qui concerne sa date de fabrication, plus récente que la deuxième installation de résonnance magnétique.

5. Procédé suivant l'une des revendications précédentes, **caractérisé**
**en ce que** la conversion (2) syntactique comprend une élimination de paramètres du protocole (1) de mesure de résonnance magnétique, qui ne sont pas supportés par l'ancienne version logicielle,
une insertion de paramètres nouveaux du protocole (1) de mesure de résonnance magnétique, qui sont supportés par l'ancienne version logicielle, mais non par la nouvelle version logicielle, une fixation des nouveaux paramètres à une valeur standard, et une prise en charge de valeurs de paramètres du protocole (1) de de mesure, qui ont, dans l'ancienne version logicielle, une dénomination autre que dans la nouvelle version logicielle.

6. Procédé suivant l'une des revendications précédentes, **caractérisé,**
**en ce que** la conversion (4) sémantique comprend :
une production d'un protocole standard de l'ancienne version logicielle, et
une copie de paramètres du protocole (1) de résonnance magnétique RM en les paramètres leur appartenant du protocole cible, dans lequel on prend en compte des limites et des dépendances des paramètres du protocole cible, en ce qui concerne l'ancienne version logicielle, et
**en ce que** le protocole cible correspond, à la fin du procédé, au protocole (6) de mesure de résonnance magnétique transformé en ce qui concerne l'ancienne version logicielle,
dans lequel on contrôle plusieurs fois tous les paramètres du protocole cible, sur le point de savoir, si des limites et des dépendances en ce qui concerne l'ancienne version logicielle ont été respectées, et
**en ce que**, dans le cas où l'un des paramètres du protocole cible enfreint l'une des limites ou l'une des dépendances, on fixe le paramètre du protocole cible de manière à ce que ce paramètre n'enfreigne plus la limite ou la dépendance,
dans lequel le stade du contrôle du point de savoir, si des limites et des dépendances sont respectées en ce qui concerne l'ancienne version logicielle et le stade de la refixation du paramètre, pour autant que le paramètre du protocole cible enfreigne l'une des limites ou l'une des dépendances, sont répétés jusqu'à ce que toutes les valeurs de paramètres respectent les limites et les dépendances en ce qui concerne l'ancienne version logicielle.

7. Système de conversion d'un protocole (1) de mesure de résonnance magnétique, qui prescrit des paramètres ayant des valeurs de paramètres leur appartenant, qui configure une mesure au moyen d'une nouvelle version logicielle d'une première installation de résonnance magnétique,
dans lequel le système (30) comprend une unité (17) de calcul et des moyens (19) de mémoire,
dans lequel le système (30) est conformé,
pour mettre le protocole (1) de mesure de résonnance magnétique, au moins en partie en mémoire, dans les moyens (19) de mémoire,
pour transformer (2) syntactiquement le protocole (1) de mesure de résonnance magnétique, au moyen de l'unité (17) de calcul, dans laquelle on adapte la structure du protocole (1) de mesure de résonnance magnétique à une version logicielle ancienne d'une deuxième installation (10) de résonnance magnétique,
afin de transformer (4) sémantiquement, au moyen de l'unité (17) de calcul, le protocole de mesure de résonnance magnétique transformé syntactiquement, en adaptant des valeurs de paramètres du protocole de mesure de résonnance magnétique transformé syntactiquement aux propriétés propres mises en oeuvre dans l'ancienne version logicielle, les dépendances entre les paramètres, y compris de leurs valeurs de paramètres, étant prises en compte.

8. Système suivant la revendication 7,
**caractérisé en ce que**
le système (30) est conformé pour effectuer le procédé suivant l'une des revendications 2 à 6.

9. Installation (10) de résonnance magnétique ayant un système (30) suivant la revendication 7 ou 8.

10. Produit de programme d'ordinateur, qui comprend un programme et qui peut être chargé directement dans une mémoire (19) d'un dispositif (20) de commande programmable d'une installation (10) de résonnance magnétique, ayant des moyens de programme pour effectuer tous les stades du procédé suivant l'une des revendications 1 à 6, lorsque le programme est exécuté dans le dispositif (20) de commande de l'installation (10) de résonnance magnétique.

11. Support de données, déchiffrables électroniquement, sur lequel sont mises en mémoire des informations de commande, déchiffrables électroniquement, qui sont conformées de manière à ce qu'elles, lors de l'utilisation du support de données dans un dispositif (20) de commande des installations (10) de résonnance, effectuent le procédé suivant l'une des revendications 1 à 6.
